# EUROPÄISCHE PATENTSCHRIFT

(11) **EP 0 393 343 B1**
(45) Veröffentlichungstag und Bekanntmachung des Hinweises auf die Patenterteilung: **14.12.1994**
(21) Anmeldenummer: 90104579.9
(22) Anmeldetag: 10.03.1990
(51) Int. Cl.: A61B 1/30

(54) **Uretero-Renoskop**
Uretero-renoscope
Endoscope pour l'examen de l'uretère et du rein

(30) Priorität: 19.04.1989 DE 3912797
(43) Veröffentlichungstag der Anmeldung: 24.10.1990
(73) Patentinhaber: Richard Wolf GmbH, 75438 Knittlingen (DE)
(72) Erfinder: Gautier, Jean-Romain, F-31500 Toulouse (FR); Bonnet, Ludwig, D-7134 Knittlingen (DE)
(74) Vertreter: Wilcken, Thomas, Dipl.-Ing.

(56) Entgegenhaltungen:
- DE-U- 8 634 860
- DE-U- 8 714 069
- GB-A- 2 170 714
- US-A- 4 557 255

## Beschreibung

Die Erfindung geht aus von einem Uretero-Renoskop nach dem Oberbegriff des Anspruches 1.

In der DE-U-86 34 860 ist ein Endoskop beschrieben, welches zur Untersuchung der Eileiter Verwendung findet und aus einem ersten Endoskop mit abgewinkelten Okularteil und aus einem zweiten Endoskop, das aus einem Außenschaft, einem Innenschaft, einer Optik und einem gegen den Innenschaft und die Optik austauschbaren Mandrin besteht. Der Innenschaft umgibt die Optik im Bereich des im Durchmesser erweiterten Abschnittes des Außenschaftes, während der den Innenschaft überragende distale Innenteil der Optik durch den im Durchmesser verengten Endteil des Außenschaftes geführt wird.

Aus dem DE-U-83 22 900 ist ein derartiges Instrument als bekannt zu entnehmen, dessen Schaft gegenüber dem proximalen Teil eine Abstufung aufweist, um einerseits durch die Durchmesserreduzierung im distalen Schaftbereich eine möglichst geringe Belastung des Ureters und andererseits durch den proximalwärts im Durchmesser erweiterten Schaft eine ausreichende mechanische Stabilität zu erzielen.

Aus der DE-PS 35 04 252 ist weiter ein Uretero-Renoskop bekannt, dessen Schaft einmal abgestuft ist und mit der Optik eine lösbare Einheit bildet,womit ermöglicht wird,daß in dem in dem Ureter verbleibenden Schaft eine zweite Optik, beispielsweise mit einer von der ersten abweichenden Blickrichtung eingesetzt werden kann.Die Abstufung des Schaftes erfolgt dabei in Anpassung an den Ureter-Durchmesser nach Maßgabe einer ausreichenden Stabilität.

Bei den Ausführungen nach dem zitierten Stand der Technik ist nachteilig, daß einerseits trotz der distal über eine bestimmte Länge erfolgten Schaftabstufungen das Einführen des Schaftes durch das Ureter-Ostium in den Ureter nicht ohne vorherige Dilatation des Ostiums und nicht ohne einen in den Instrumentenkanal eingeführten Obturator erfolgen kann und andererseits durch die Dilatation die Zeit des endoskopischen Eingriffes in ganz erheblichem Umfang verlängert wird, insbesondere aber auch Traumatisierungen der Ostien aufgrund der erforderlichen Dilatation nicht auszuschließen sind.Ein weiterer Nachteil des Standes der Technik besteht darin, daß anhand des zur Verfügung stehenden freien Ureterlumens optimierte ovale Instrumentenquerschnitt nur für die Durchführung von Instrumenten mit einem relativ geringen runden Querschnitt verwendet werden kann, so daß unter Umständen ein erheblicher Teil des freien Lumens nicht benutzt oder Instrumente, die aufgrund besonderer Anwendungsweise einen größeren Außendurchmesser aufweisen, in Verbindung mit den Schäften nach dem Stand der Technik , keine Verwendung finden können.

Es ist daher die Aufgabe der Erfindung, ein Uretero-Renoskop anzugeben, das ohne Verwendung eines zusätzlichen Dilatators und Obturators in und durch das Ostium in den Ureter eingeführt und dieser Vorgang sowie das weitere Einführen des Uretero-Renoskops ggf. bis zum Nierenbecken und den oberen Kelchgruppen unter ständiger direkter visueller Kontrolle durchgeführt werden kann.Des weiteren soll ermöglicht werden, daß das Uretero-Renoskop bei Bedarf gegen ein anderes Instrument auswechselbar ist, das das freie Lumen des im Ureter verbleibenden zusätzlichen Schaftes vollständig nutzt.

Diese Aufgabe wird erfindungsgemäß durch die Merkmale des Patentanspruchs 1 gelöst.

Die damit erzielbaren Vorteile bestehen insbesondere darin, daß aufgrund des geringen Querschnittes des distalseitigen Uretero-Renoskopschaftendes dieses problemlos in das Ostium hinein und durch dieses hindurch in den Ureter ohne eine durch das Hochschieben des Ureters verursachte Schleifenbildung eingeführt werden kann. Durch die mehrfache Abstufung des distalen Instrumentenendes ist weiterhin gewährleistet, daß bei gleichzeitiger visueller Kontrolle durch das optische System hindurch eine sanfte und nicht abrupte und insbesondere eine das Ostium nicht traumatisierende Dilatation erfolgt.

Durch die erfindungsgemäße Ausbildung des Uretero-Renoskops gleichzeitig als Dilatator ist es in vorteilhafter Weise nicht erforderlich, das Ostium aufgrund seiner Rückstellneigung über das erforderliche Maß hinaus dilatieren oder in diesem einen den Öffnungsquerschnitt beibehaltenden Katheter oder dergleichen belassen zu müssen.

Durch die Erfindung ist es somit erstmals möglich, die schonende und das Ostium nicht traumatisierende Dilatation durchzuführen sowie im unmittelbaren Anschluß daran eine visuelle Kontrolle des Ureters bzw. den unter Umständen erforderlichen endoskopischen Eingriff vornehmen zu können. Eine weitere Bedeutung ist der Tatsache beizumessen, daß der Patient durch die geringere Behandlungszeit einer in einem nicht zu unterschätzenden Maße geringeren Belastungen sowie einem verminderten Infektionsrisiko unterliegt.

Durch den auf dem proximalwärts letzten Schaftabschnitt aufgeschobenen Rohrschaft wird eine Steigerung der mechanischen Stabilität des Renoskopschaftes erreicht, wobei dieser Rohrschaft gleichzeitig als Dilatator fungiert, über den zusätzlich Spülflüssigkeit zu- und abgeführt werden kann, und der aufgrund seiner lösbaren Arretierbarkeit auf dem Renoskopschaft im Bedarfsfall bei einer Entnahme des Uretero-Renoskops zur Durchführung weiterer Therapien benutzt werden kann, indem beispielsweise ein Ureterotom zur Schlitzung einer Ureter-Stenose in den Ureter eingeführt wird.

Das erfindungsgemäße Uretero-Renoskop ist nachfolgend anhand eines in der Zeichnung dargestellten Ausführungsbeispiels näher erläutert.

Es zeigen:
- Figur 1: das erfindungsgemäße Uretero-Renoskop in einer Seitenansicht ohne den zusätzlich aufschiebbaren Rohrschaft 5,
- Figur 2: eine Ansicht des mehrfach abgestuften Schaftendes nach Figur 1,
- Figur 3: eine Draufsicht auf das distale Schaftende in Richtung des Pfeiles III in Fig. 2 in vergrößertem Maßstab.

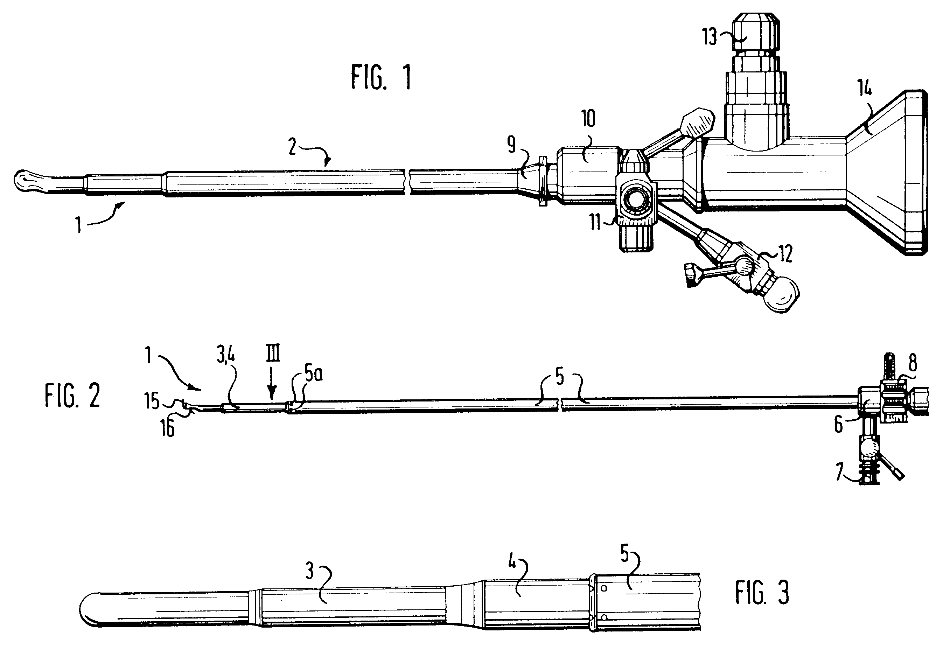

Das in den Figuren 1 und 2 dargestellte Uretero-Renoskop 1 besteht im wesentlichen aus einem langen, relativ dünnen Schaft 2 ,der insbesondere in seinem distalen Bereich mehrere aufeinanderfolgende Schaftabstufungen 3 und 4 aufweist. Dabei ist der distale Schaftabschnitt an seiner Stirnseite atraumatisch ausgebildet und weist einen ovalen Querschnitt auf, an den sich proximalwärts der Schaftabschnitt 3 mit ebenfalls ovalem Querschnitt anschließt, der dann in den Schaftabschnitt 4 übergeht der einen runden Querschnitt aufweist.Auf diesem Schaftabschnitt 4 ist ein Rohrschaft 5 aufgeschoben, der proximalseitig ein Kupplungsdrehteil 6 mit einem Abschlußhahn 7 sowie in seinem distalen Endbereich mehrere Öffnungen 5a zur Zu- und/oder Abführung einer Spülflüssigkeit oder dergleichen aufweist. Das Kupplungsdrehteil 6 ist mittels eines Spannringes 8 in einem Kupplungskegel 9 lösbar befestigbar,der an einem an dem proximalen Ende des Schaftabschnittes 4 befindlichen Gehäuse 10 angeordnet ist. Das Gehäuse 10 ist mit einem oder mehreren Anschlußhähnen 11, einem im Winkel zur Instrumentenlängsachse schräg angeordneten Einführungshahn 12, einem Anschluß 13 zur Aufnahme eines Lichtleitkabelanschlußteiles versehen und dient der Aufnahme eines Okulars mit einem Okulartrichter 14 eines nicht dargestellten optischen Bildübertragungssystems.

Wie aus Fig. 2 zu ersehen ist, befindet sich an dem stirnseitigen Ende des Schaftes 2 ein Ausblickfenster 15 für ein in dem Schaft 2 integriertes optisches System sowie das Austrittsende eines diesen durchsetzenden, nicht weiter dargestellten Kanals 16 für die Einführung der Instrumente und sonstigen Arbeitsmittel.

Aufgrund der Abstufungen 3 und 4, die in Abständen von 5 bis 50 mm aufeinanderfolgen können, kann das Uretero-Renoskop ohne vorherige, zusätzlich vorzunehmende Dilatation und ohne zusätzlichen Obturator durch das Ostium hindurch in den Ureter eingeführt werden. Andererseits besteht die Möglichkeit, auf dem sich proximalwärts erstreckenden Schaft 2 mit rundem Querschnitt einen zusätzlichen, rund ausgebildeten Schaft 5 lösbar festzulegen, der neben der erwünschten Stabilitätssteigerung im Bedarfsfall zur Einführung eines anderen Instrumentes, nach der Entnahme des Uretero-Renoskops 1 im Ureter verbleiben kann.

## Patentansprüche

1. Uretero-Renoskop, das einen Schaft (2) mit einem Instrumentenkanal zum Hindurchführen von Hilfsinstrumenten und mit einem inneren optischen System, ein sich an den Schaft anschließendes Optikgehäuse (10) für den Anschluß eines Lichtleiterkabels für das optische System sowie Anschlüsse (11, 12) für das Zu- und Abführen einer Spülflüssigkeit aufweist, wobei der Schaft (2), insbesondere in seinem distalen Längenbereich, mehrere aufeinanderfolgende, in proximaler Richtung querschnittserweiternde Abstufungen (3, 4) aufweist und im Bereich seines distalen Schaftendes mit einem ovalen Querschnitt ausgeführt ist, der in Abhängigkeit von dem Durchmesser des genannten optischen Systemes und einem zusätzlichen Instrumentenkanal kleinstmöglichen Durchmessers dimensioniert ist, und auf der proximalwärts letzten, einen runden Querschnitt aufweisenden Schaftabstufung ein eine weitere Abstufung bildender, mit einem Kupplungskegel (9) des proximalseitigen Optikgehäuses (10) kuppelbarer Rohrschaft (5) lösbar festlegbar ist.

2. Uretero-Renoskop nach Anspruch 1, dadurch gekennzeichnet, daß der Rohrschaft (5) für die lösbare Arretierung proximalseitig mit einem Spannring (8) versehen ist, der auf dem Kupplungskegel (9) des Optikgehäuses (10) lösbar festlegbar ist.

3. Uretero-Renoskop nach Anspruch 1 oder 2, dadurch gekennzeichnet, daß der Rohrschaft (5) proximalseitig mit Anschlußmitteln (7) und in seinem distalen Endbereich mit mehreren Öffnungen (5a) für die Zu- und/oder Abführung einer Spülflüssigkeit versehen ist.

4. Uretero-Renoskop nach Anspruch 1, 2 oder 3, dadurch gekennzeichnet, daß die einzelnen Abstufungen in Abständen von 5 bis 50 mm aufeinanderfolgen.

## Claims

1. Uretero-renoscope which has a shaft (2) with an instrument channel for feeding through auxiliary instruments and with an inner optical system, a lens housing (10) adjoining the shaft for the connection of a fibre optic cable for the optical system as well as connections (11, 12) for the supply and removal of a flushing liquid, wherein the shaft (2), in particular in its distal length region, has several successive graduations (3, 4) which increase the cross-section in the proximal direction and is designed in the region of its distal shaft end with an oval cross-section which is dimensioned with the minimum possible diameter as a function of the diameter of the said optical system and an additional instrument channel, and on the last shaft graduation in the proximal direction, which has a round cross-section, a pipe shaft (5) can be releasably fixed which forms a further graduation and can be coupled to a coupling cone (9) of the lens housing (10) on the proximal side.

2. Uretero-renoscope according to claim 1, characterised in that the pipe shaft (5) for releasable locking is provided on the proximal side with a clamping ring (8) which can be fixed releasably on the coupling cone (9) of the lens housing (10).

3. Uretero-renoscope according to claim 1 or 2, characterised in that the pipe shaft (5) is provided on the proximal side with connecting means (7) and in its distal end region with several openings (5a) for the supply and/or removal of a flushing liquid.

4. Uretero-renoscope according to claim 1, 2 or 3, characterised in that the individual graduations succeed each other at intervals of 5 to 50 mm.

## Revendications

1. Endoscope urétro-rénal comprenant un corpstige (2) renfermant un canal d'instrument pour le guidage traversant d'instruments auxiliaires, et un système optique interne, un boîtier d'optique (10) se raccordant au corps-tige et destiné au raccordement d'un câble guide-lumière pour le système optique, ainsi que des raccords (11, 12) pour l'amenée et l'évacuation d'un liquide de rinçage, le corps-tige (2) comportant, notamment dans la zone distale de son étendue en longueur, plusieurs étagements successifs (3,4) dont la section transversale s'agrandit en direction proximale, et présentant dans la zone de l'extrémité distale du corps-tige une section transversale de forme ovale, dimensionnée en fonction du diamètre du système optique cité et d'un canal d'instruments supplémentaire de diamètre le plus faible possible, et un corps-tige tubulaire (5) qui forme un autre étagement et qui peut être couplé à un cône d'accouplement (9) du boîtier d'optique (10) situé côté proximal, étant susceptible d'être fixé de manière amovible sur le dernier étagement côté proximal, qui présente une section transversale ronde.

2. Endoscope urétro-rénal selon la revendication 1, caractérisé en ce que le corps-tige tubulaire (5) est pourvu, côté proximal, pour le verrouillage amovible, d'une bague de contrainte (8) qui peut être fixée de manière amovible sur le cône d'accouplement (9) du boîtier d'optique (10).

3. Endoscope urétro-rénal selon la revendication 1 ou 2, caractérisé en ce que le corpstige tubulaire (5) est pourvu, côté proximal, de moyens de raccordement (7), et dans sa zone d'extrémité distale, de plusieurs ouvertures (5a) pour l'alimentation et/ou l'évacuation d'un liquide de rinçage.

4. Endoscope urétro-rénal selon la revendication 1, 2 ou 3, caractérisé en ce que les étagements individuels se succèdent à des distances de 5 à 50 mm.
